(19) Europäisches Patentamt
European Patent Office
Office européen des brevets

(11) EP 1 194 932 B1

(12) # FASCICULE DE BREVET EUROPEEN

(45) Date de publication et mention de la délivrance du brevet:
**28.04.2004 Bulletin 2004/18**

(21) Numéro de dépôt: **00951662.6**

(22) Date de dépôt: **12.07.2000**

(51) Int Cl.$^7$: **H01B 1/12**

(86) Numéro de dépôt international:
**PCT/FR2000/002017**

(87) Numéro de publication internationale:
**WO 2001/004910 (18.01.2001 Gazette 2001/03)**

(54) **UTILISATION D'ACIDES SULFONIQUES ET PHOSPHONIQUES COMME DOPANTS DE FILMS CONDUCTEURS DE POLYANILINE ET DE MATERIAUX COMPOSITES CONDUCTEURS A BASE DE POLYANILINE**

VERWENDUNG VON SULFON- UND PHOSPHONSÄUREN ALS DOTIERSTOFFE VON POLYANILINFILMEN UND AUS POLYANILIN LEITENDE VERBUNDWERKSTOFFE

USE OF SULPHONIC AND PHOSPHONIC ACIDS AS DOPANTS OF CONDUCTIVE POLYANILINE FILMS AND CONDUCTIVE COMPOSITE MATERIALS BASED ON POLYANILINE

(84) Etats contractants désignés:
**DE FI GB NL**

(30) Priorité: **13.07.1999 FR 9909088**

(43) Date de publication de la demande:
**10.04.2002 Bulletin 2002/15**

(73) Titulaires:
- **Commissariat à l'Energie Atomique
75752 Paris Cedex 15 (FR)**
- **CENTRE NATIONAL DE
LA RECHERCHE SCIENTIFIQUE (CNRS)
75794 Paris Cedex 16 (FR)**

(72) Inventeurs:
- **OLINGA, Thomas
F-38000 Grenoble (FR)**
- **PRON, Adam
F-38100 Grenoble (FR)**
- **TRAVERS, Jean-Pierre
F-38400 Saint-Martin D'Hères (FR)**

(74) Mandataire: **Audier, Philippe André et al
Brevalex,
3, rue du Docteur Lancereaux
75008 Paris (FR)**

(56) Documents cités:
**WO-A-98/05040** **US-A- 5 783 111**

- **Y. CAO, ET AL.: "Counter-ion induced processibility of conducting polyaniline and of conducting polyblends of polyaniline in bulk polymers" SYNTHETIC METALS, vol. 48, 1992, pages 91-97, XP000195647 Elsevier Sequoia, Lausanne, CH ISSN: 0379-6779 cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 194 932 B1

Printed by Jouve, 75001 PARIS (FR)

## Description

### Domaine technique

La présente invention concerne de l'utilisation d'acides sulfoniques et phosphoniques comme dopants de matériaux conducteurs de l'électricité à base de polyaniline.

De façon plus précise, elle concerne la fabrication de films de polyaniline hautement conducteurs, ayant de bonnes propriétés mécaniques et pouvant être utilisés soit sous la forme de films minces déposés sur un substrat approprié, soit sous la forme de films auto-supportés.

Elle concerne également la fabrication de films de matériaux composites conducteurs à base de polyaniline et de polymère isolant.

Ces films de polyaniline et de matériaux à base de polyaniline peuvent être utilisés dans le blindage électromagnétique, comme matériaux antistatiques ou comme électrodes semi-transparentes dans des dispositifs optoélectroniques.

### État de la technique antérieure

Généralement, on prépare des films de polyaniline conducteurs à partir d'une solution de polyaniline dans un solvant organique par coulée de la solution et évaporation du solvant. Pour obtenir un film conducteur, il est nécessaire que la polyaniline soit conductrice. Or, la polyaniline est généralement obtenue dans sa forme basique qui n'est pas conductrice, il est donc nécessaire de convertir cette forme basique en une forme protonée conductrice, soit avant la préparation du film, soit après sa fabrication.

Cette conversion peut être obtenue par un agent dopant approprié, généralement par un acide pour faire subir à la polyaniline une réaction de protonation.

La protonation de la polyaniline, après obtention du film, est une complication difficilement acceptable sur le plan industriel. De plus, il est difficile d'obtenir dans ce cas un produit homogène puisque la protonation est effectuée à l'état solide.

Un autre problème rencontré pour la préparation de films de polyaniline réside dans le fait que la polyaniline est un polymère à la fois infusible et très difficile à dissoudre.

Le document [1] : Synthetic Metals, 21, 1987, pp. 21-30, illustre l'emploi d'acide acétique pour former à partir d'une polyaniline sous forme éméraldine, une solution de polyaniline protonée qui peut conduire à la formation de films de polyaniline par évaporation du solvant.

Cependant les films obtenus à partir de ces solutions ont une faible conductivité (0,5 à 2 S/cm) et de très mauvaises propriétés mécaniques. De plus, ils résistent mal à la déprotonation, ce qui entraîne une forte décroissance de la conductivité du polymère.

La référence [2] : Polymer, 34, n°20, 1993, pp. 4235-4240, illustre l'utilisation de diesters d'acides phosphoriques pour protoner une polyaniline sous forme éméraldine et la rendre soluble dans divers solvants tels que le toluène, le chloroforme et le tétrahydrofurane (THF) en vue de préparer des films de polyaniline conducteurs. Dans ce cas, l'agent protonant agit simultanément comme plastifiant et facilite ainsi la mise en oeuvre de polyanilines sous forme de films ou sous forme de matériaux composites.

La référence [3] : Synthetic Metals, 48, 1992, pp. 91-97, illustre l'utilisation d'un autre agent de protonation de la polyaniline constitué par l'acide dodécylbenzènesulfonique. Comme précédemment l'emploi de cet agent de protonation facilite la mise en oeuvre de la polyaniline sous forme de films. Bien que les films obtenus conformément aux références [2] et [3] soient plus résistants à la déprotonation, ils possèdent des propriétés mécaniques médiocres et une conductivité moyenne.

La référence [4] : Kulszewicz-Bajer et al, Synthetic Metals, 101, 1999, pp. 713-714, illustre l'utilisation des diesters di(n-amyle), di(n-décyle), di(butoxy-2-éthyle) et di[2-(butoxy-2-éthoxy)éthyle] de l'acide 5-sulfoisophtalique ainsi que les esters de rocanol de cet acide 5-sulfoisophtalique comme dopants d'une polyaniline. Cependant, les films de polyaniline dopés avec ces esters obtenus à partir d'une solution dans le chloroforme ont des conductivités très insuffisantes, de l'ordre de $3.10^{-3}$ S/cm.

On peut obtenir des films présentant une conductivité élevée, excédant par exemple 300 S/cm, par évaporation d'une solution comprenant une polyaniline, de l'acide camphre sulfonique comme dopant et du méta-crésol comme solvant, comme il est décrit dans J. Chem. Phys., 103, 22, 1995, pp. 9855-9863 [5].

Cette solution qui paraît intéressante, présente l'inconvénient que les films contiennent 12 à 14 % en poids de méta-crésol résiduel qui est un produit toxique. Un autre système de dopant et de solvant tel que le système acide de 4-éthylbenzène sulfonique - hexafluoropropanol, décrit dans la référence [6] : Polymer Preprints, 36, 1995, pp. 396-397, conduit à un film hautement conducteur, mais l'emploi de cet alcool fluoré à l'échelle industrielle n'est pas possible à cause de sa toxicité et de son coût élevé.

La référence [7] : J. Phys. : Condens. Matter, 10, 1998, pp. 8293-8303, décrit l'emploi du système acide 2-acrylamido-2-méthyl-1-propane sulfonique - acide dichloroacétique, qui permet d'obtenir une conductivité élevée. Cependant, les films obtenus possèdent de faibles propriétés mécaniques.

Ainsi, aucun des systèmes décrits ci-dessus acceptables pour une mise en oeuvre à l'échelle industrielle ne permet d'obtenir simultanément un film de polyaniline, ayant une conductivité et des propriétés mécaniques élevées, notamment une bonne flexibilité.

Le problème se pose également d'obtenir des matériaux composites conducteurs à partir d'un mélange de polyaniline avec des polymères isolants, ayant à la fois de bonnes propriétés conductrices et de bonnes propriétés mécaniques.

La référence [8] : Physical Review B, 50, 1994, pp. 13931-13941, et la référence [9] : WO-A-98/05040, décrivent la fabrication de composites conducteurs comprenant une matrice hôte polymère dans laquelle est répartie une polyaniline conductrice, présentant des seuils de percolation extrêmement bas, inférieurs à 1 %.

Le seuil de percolation peut être défini comme la fraction volumique minimale de phase conductrice qui rend le matériau conducteur à l'échelle macroscopique.

Ce seuil de percolation peut être déterminé à partir de la formule suivante :

$$\sigma(f) = c(f-f_c)^t$$

dans laquelle :

- $\sigma$ représente la conductivité,
- c est une constante,
- t est l'exposant critique,
- f représente la fraction volumique de la phase conductrice, et
- $f_c$ est la fraction de la phase conductrice au seuil de percolation.

Dans des composites à faible seuil de percolation, on pourrait penser que les propriétés mécaniques de la phase conductrice ne devraient pas influer beaucoup sur les propriétés mécaniques du composite puisque cette fraction est très faible.

Cependant, on constate que les propriétés mécaniques du composite sont détériorées par la présence de la phase conductrice de polyaniline, même dans des systèmes ayant un très faible seuil de percolation. Par conséquent, il est d'un grand intérêt d'améliorer les propriétés mécaniques de la polyaniline dans de tels composites.

## Exposé de l'invention

La présente invention a précisément pour objet l'utilisation de nouveaux agents dopants des polyanilines, qui permettent d'améliorer les propriétés mécaniques de films de polyaniline dopés par ces agents. Ces nouveaux dopants agissent à la fois comme plastifiant et comme agent protonant. Ils permettent l'obtention de conductivités élevées jusqu'à environ 200 à 300 S/cm de type métallique et une amélioration concomitante des propriétés mécaniques des films de polyanilines et des composites conducteurs à base de polyaniline avec des polymères isolants, au voisinage et au-dessus du seuil de percolation.

Ainsi, l'invention a pour objet une composition pour la fabrication de films de polyaniline, constituée par une solution dans un solvant organique d'une polyaniline sous la forme éméraldine base et d'un dopant constitué par un acide sulfonique ou phosphonique répondant à la formule :

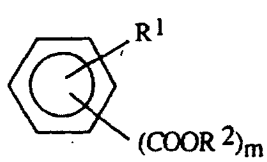 (I)

dans laquelle :

- $R^1$ représente $-SO_3H$ ou $PO_3H_2$,
- $R^2$ est un groupe alkyle linéaire ou ramifié et m est égal à 1 ou 2, ou

- $R^2$ est un groupe de formule :

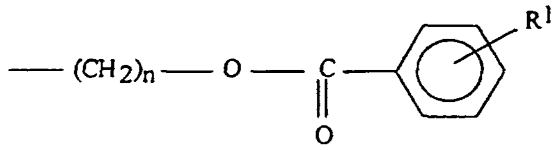

(II)

dans laquelle $R^1$ est tel que défini ci-dessus et n est un nombre entier allant de 1 à 16, et m est égal à 1, à l'exception des esters di(n-anyle), di(n-décyle), di(butoxy-2-éthyle) et di-2-(butoxy-2-éthoxy)éthyle de l'acide 5-sulfoisophtalique et des esters de l'acide 5-sulfoisophtalique et de rocanol.

Elle concerne également une composition pour la fabrication d'un matériau composite conducteur comprenant :

- un solvant organique,
- une polyaniline sous la forme éméraldine base,
- un dopant constitué par un acide sulfonique ou phosphonique répondant à la formule :

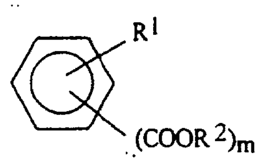

(I)

dans laquelle :

- $R^1$ représente $-SO_3H$ ou $PO_3H_2$,
- $R^2$ est un groupe alkyle linéaire ou ramifié et m est égal à 1 ou 2, ou
- $R^2$ est un groupe de formule :

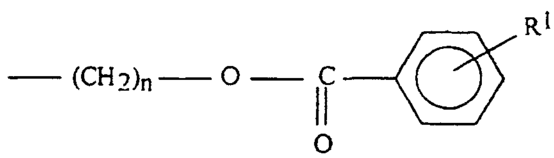

(II)

dans laquelle $R^1$ est tel que défini ci-dessus et n est un nombre entier allant de 1 à 16, et m est égal à 1,

- un polymère isolant, et
- un plastifiant du polymère isolant.

Dans la formule (I), le groupe $R^2$ est un groupe alkyle, linéaire ou ramifié, ayant de préférence de 4 à 12 atomes de carbone de façon à former un groupe hydrophobe jouant le rôle de plastifiant et permettant d'améliorer la solubilité des polyanilines dans des solvants organiques.

A titre d'exemple de groupe susceptible d'être utilisé, on peut citer par exemple le groupe 2-éthyl-hexyle.

Dans le cas où m est égal à 2, ces acides phosphoniques ou sulfoniques correspondent à des esters d'acides phtalique, isophtalique et téréphtalique fonctionnalisés avec un groupement acide du type $-SO_3H$ ou $-PO_3H_2$.

Dans le cas où m est égal à 1, il s'agit de benzoates fonctionnalisés sur le cycle aromatique par les groupements acides précités ou de dibenzoates fonctionnalisés également par des groupements acides et répondant à la formule :

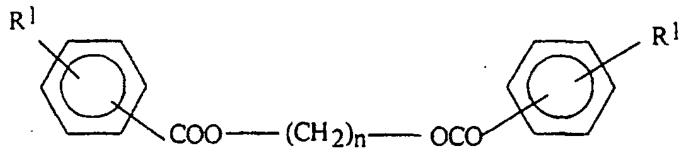

A titre d'exemple, l'acide sulfonique peut répondre à la formule :

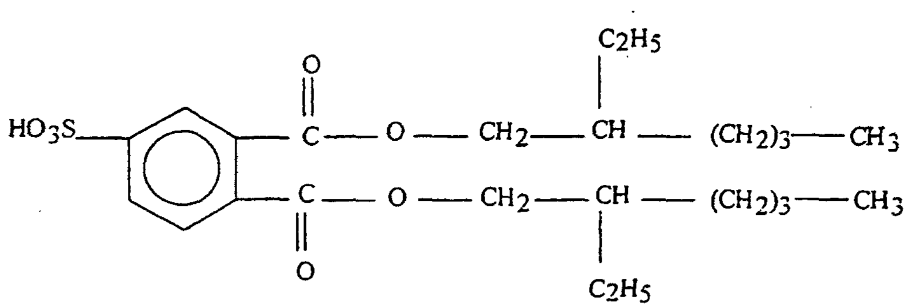 (III)

Ces acides sulfoniques ou phosphoniques peuvent être préparés à partir des acides phtaliques ou benzéniques fonctionnalisés correspondants répondant à la formule :

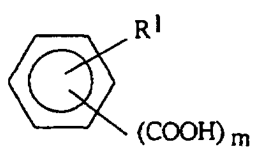 (IV)

dans laquelle $R^1$ et m sont tels que définis ci-dessus par estérification de ces acides au moyen d'un alcool de formule $R^2OH$ dans laquelle $R^2$ est tel que défini ci-dessus.

Les acides de formule (IV) utilisés comme produits de départ dans ce procédé sont des produits du commerce ou peuvent être préparés par sulfonation ou phosphorylation des acides phtaliques ou de l'acide benzénique.

Dans le cas où $R^2$ représente le groupe de formule (II) et m est égal à 1, on peut préparer l'acide sulfonique ou phosphonique de la même façon par estérification de l'acide de formule (IV) avec l'alcool correspondant $R^2OH$ où $R^2$ est le groupe de formule (II), soit alternativement par condensation de deux molécules de formule (IV) avec m = 1, avec un diol de type HO- $(CH_2)_n$-OH.

Les acides sulfoniques et phosphoniques décrits ci-dessus présentent de bonnes propriétés comme agent protonant en raison du groupe $SO_3H$ ou $PO_3H_2$, et comme plastifiant et solubilisant grâce à la présence du groupe :

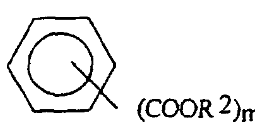

Ainsi, ils peuvent être mis en oeuvre dans des compositions pour la fabrication de films de polyaniline.

Dans les compositions de l'invention, le rôle du dopant fonctionnalisé de formule (I) est d'améliorer les propriétés mécaniques et plus particulièrement la flexibilité des films ou des dépôts obtenus à partir de la composition par évaporation du solvant.

La polyaniline utilisée dans cette composition est sous forme d'éméraldine base. Elle peut être préparée par polymérisation oxydative classique ou par polymérisation enzymatique ou par d'autres procédés tels que la polymérisation électrochimique. L'éméraldine base répond à la formule :

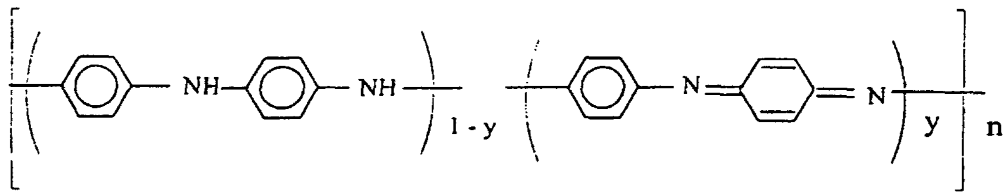 (I)

avec y = 0,5.

On précise que selon l'invention, on entend par polyaniline non seulement les polyanilines obtenues par polymérisation de l'aniline, mais également les polyanilines obtenues à partir d'aniline substituée sur le cycle ou sur l'azote.

Les solvants utilisés dans ces compositions peuvent être de divers types, mais on préfère utiliser des solvants non toxiques et peu coûteux qui n'ont pas tendance à rester dans la matrice du film de polyaniline après coulée et évaporation du solvant.

Aussi, on utilise de préférence comme solvant, des dérivés halogénés d'un acide carboxylique de formule :

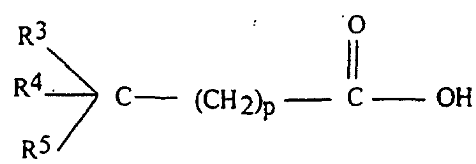 (V)

dans laquelle $R^3$, $R^4$ et $R^5$ qui peuvent être identiques ou différents, représentent H ou un atome d'halogène choisi parmi F, Cl et Br, au moins l'un des $R^3$, $R^4$ et $R^5$ représentant un atome d'halogène, et p est égal à 0,1 ou 2.

Lorsque les dérivés répondant à la formule (V) comprennent 2 ou 3 atomes d'halogènes, ceux-ci peuvent bien entendu être différents.

A titre d'exemple de solvants utilisables, on peut citer les acides dichloroacétique, difluoroacétique, α-trifluoroacétique, chlorodifluoracétique, 2-chloropropionique, 2-bromobutyrique et 2,2-dichloro-propionique.

Dans les compositions de l'invention pour la fabrication de films de polyaniline conducteurs, les teneurs en polyaniline et en dopant de la solution sont telles que le rapport molaire du dopant à la polyaniline sous la forme éméraldine base est dans la gamme de 0,4 à 0,6.

Ce rapport est calculé sur la base d'une masse molaire de l'éméraldine base correspondant à un motif monomère de l'éméraldine qui est de 90,5 g. La teneur en polyaniline de la solution est généralement située dans la gamme de 0,1 à 1 % en poids.

Dans le cas où l'on utilise des concentrations élevées, le temps de stockage de la solution doit être réduit pour éviter la gélification.

Pour préparer un film de polyaniline à partir de cette composition, on coule la solution sur un support et on procède à l'évaporation du solvant. Le support peut être laissé ou on peut fabriquer un film auto-supporté en le séparant du support.

L'invention a également pour objet une composition pour la fabrication de matériaux composites conducteurs à base de polyaniline, qui comprend :

- un solvant organique,
- une polyaniline sous la forme éméraldine base,
- un dopant constitué par un acide sulfonique ou phosphonique de formule (I) décrit ci-dessus,
- un polymère isolant, et
- un plastifiant du polymère isolant.

Dans cette composition, les dopants et les solvants organiques utilisés sont identiques à ceux cités dans le cas de la composition pour la fabrication de films de polyaniline.

Les concentrations en polyaniline et en dopant dans la solution sont également situées dans les mêmes gammes.

Dans cette composition, le rôle du dopant fonctionnalisé est non seulement d'améliorer les propriétés mécaniques, notamment la flexibilité, mais aussi d'abaisser le seuil de percolation du matériau composite à base de polyaniline et de polymère isolant.

Les polymères isolants utilisés peuvent être de différents types. A titre d'exemple, on peut citer le polystyrène, le poly(métacrylate de méthyle), les polymères cellulosiques, les poly(chlorure de vinyle), les polycarbonates, les polyesters et les polyuréthanes.

Dans cette composition, on utilise également un plastifiant du polymère isolant. On peut utiliser des plastifiants classiques tels que les diesters des acides phtaliques, les diesters d'acides dicarboxyliques et les triesters d'acide phosphorique.

Le choix de ces plastifiants permet encore d'abaisser le seuil de percolation du matériau composite.

Les teneurs en plastifiant et en polymère isolant de la composition sont choisies de façon à obtenir une conductivité satisfaisante du matériau composite.

Généralement, on prépare la composition en mélangeant une première solution de la polyaniline et du dopant dans le solvant à une deuxième solution dans le même solvant du polymère isolant et du plastifiant.

La concentration de la première solution en polyaniline et en dopant est généralement de 0,1 à 1 % en poids.

La concentration de la deuxième solution en polymère isolant et en plastifiant est généralement de 5 à 10 % en poids et le rapport de la teneur en plastifiant sur la teneur en polymère isolant est généralement de 20 à 45 % en poids.

Pour préparer la composition, on mélange les deux solutions pendant un temps suffisant pour obtenir une bonne homogénéité.

Pour préparer le matériau composite, on coule ensuite ladite composition pour former un film et on évapore lentement le solvant.

L'évaporation peut être réalisée à la température ambiante ou à une température supérieure, par exemple de 25 à 40°C, sous atmosphère d'azote.

Généralement, les quantités de solution mélangées sont telles que l'on obtient, après évaporation du solvant, un matériau composite comprenant :

a) 0,06 à 10 % en poids de polyaniline et de dopant,
b) 55 à 99,9 % en poids de polymère isolant, et,
c) jusqu'à 44,94 % en poids de plastifiant du polymère isolant.

D'autres caractéristiques et avantages de l'invention apparaîtront mieux à la lecture des exemples suivants donnés bien entendu à titre illustratif et non limitatif, en référence aux dessins annexés.

## Brève description des dessins

La figure 1 illustre les variations des conductivités réduites d'un film de polyaniline conforme à l'invention PANI(DEHEPSA)$_{0,5}$/DCAA et d'un film de polyaniline de l'art antérieur PANI(CSA)$_{0,5}$/MC en fonction de la température (en K).

La figure 2 illustre les courbes contrainte/allongement d'un film de polyaniline conforme à l'invention PANI (DEHEPSA)$_{0,5}$/DCAA et d'un d'un film de polyaniline de l'art antérieur PANI(CSA)$_{0,5}$/MC.

## Exposé détaillé des modes de réalisation

Les exemples qui suivent illustrent l'emploi de l'ester di-2-éthylhexylique de l'acide 1,2-benzène dicarboxylique-4-sulpho, c'est-à-dire l'acide sulfonique de formule (III) comme dopant pour la fabrication de films de polyaniline et de films de matériau composite à base de polyaniline.

### Exemple 1

Dans cet exemple, on prépare un film auto-supporté hautement conducteur de polyaniline conforme à l'invention, qui possède d'excellentes propriétés mécaniques, notamment de flexibilité. Le dopant utilisé est l'ester di-2-éthylhexylique de l'acide 1,2-benzène dicarboxylique-4-sulpho, c'est-à-dire l'acide sulfonique de formule (III).

Ce dopant permet de rendre conductrice la polyaniline éméraldine base.

a) Préparation de l'éméraldine base.

On prépare l'éméraldine base selon une version modifiée du procédé décrit dans la publication de P.M. Beadle, Y. F. Nicolau, E. Banka, P. Rannou et D. Djurado dans Synthetic Metals, 95, 1998, pp. 29-45 [10]. On effectue la réaction à -27°C.

Premièrement une solution d'aniline est préparée dans un mélange eau-alcool-HCl et LiCl. La composition exacte est la suivante : 10 ml (0,1097 mole) d'aniline, 85 ml de solution 3M HCl, 95 ml d'éthanol et 16 g de LiCl. A cette solution, on ajoute la solution contenant l'oxydant, préalablement refroidie à -27°C, dont la composition est la suivante : 6,25 g (0,0274 mole) de persulfate d'ammonium, 60 ml de solution 2M HCl et 8 g de LiCl. On effectue la réaction en mesurant en permanence la température et le potentiel de la solution. Après environ 2 heures, on ajoute une solution réductrice, composée de 3,64 g (0,0183 mole) de FeCl2, 5 g de LiCl et 50 ml de HCl 2M pour mieux contrôler l'état d'oxydation de la polyaniline. Après 1 heure supplémentaire, on arrête la réaction en séparant le polymère précipité, soit par centrifugation, soit par filtration. On lave ensuite plusieurs fois le précipité à l'eau distillée, puis au méthanol et finalement on le sèche jusqu'à l'obtention d'une masse constante. Le sel d'éméraldine ainsi obtenu est ensuite transformé en éméraldine base par traitement dans 2 litres de solution aqueuse de $NH_3$, 0,3M pendant 48 heures. On rince ensuite l'éméraldine base dans 5 à 6 litres d'eau distillée, puis dans 2 litres de méthanol, puis on la sèche jusqu'à obtention d'une masse constante.

Dans l'étape suivante, on élimine les fractions de faible poids moléculaire par des extractions successives au méthanol et au chloroforme dans un Soxhlet. La viscosité inhérente de l'éméraldine base ainsi obtenue en solution à 0,1 % en poids dans l'acide sulfurique à 98 % est de 2,25 dl/g.

b) Préparation de l'ester di-2-éthylhexylique de l'acide 1,2-benzène dicarboxylique-4-sulpho.

On mélange 6,3 g (25,6 mmole) d'acide 4-sulfophtalique sous forme de solution aqueuse à 50 % en poids, à 10 g (76,8 mmole) de 2-éthyl-1-hexanol à température ambiante, sous un flux d'azote. On élève la température du mélange à 110 - 120°C. La réaction est poursuivie à cette température pendant 2 à 3 heures en éliminant constamment l'eau produite dans la réaction par distillation. On verse ensuite le mélange réactionnel dans une solution aqueuse de HCl 1M. Après 5 à 10 min, le mélange se sépare en deux phases. On recueille la phase organique, qui est lavée 3 fois avec une solution aqueuse de HCl 1M. Le produit est ensuite extrait à l'éther. Après évaporation de l'éther et des impuretés volatiles, le produit est séché sur MgSO4. Finalement, le produit est identifié et caractérisé par analyse élémentaire, spectroscopie IR, spectroscopie de masse et RMN du proton.

c) préparation du film auto-supporté et hautement conducteur de la polyaniline.

On mélange 140 mg de polyaniline base et 369 mg de l'ester di-2-éthylhexylique de l'acide 1,2-benzène. dicarboxylique-4-sulpho (DEHEPSA) avec 48 ml d'acide dichloroacétique (DCAA). On agite vigoureusement le mélange à température ambiante pendant 4 à 5 jours jusqu'à ce qu'on n'observe plus d'évolution du spectre UV-Vis-PIR. La solution formée est filtrée sur un microfiltre de 0,45 µm. On coule un film à partir de cette solution par évaporation à 40°C sous flux d'azote. On rince le film obtenu à l'eau et on le sèche sous vide à 50°C. Le film possède une excellente flexibilité : il peut être plié plusieurs fois sans aucune dégradation. Les analyses élémentaires montrent que le film ne contient plus de solvant. Sa conductivité mesurée par une méthode à 4 contacts est typiquement de 100 à 200 S/cm à température ambiante.

Sur la figure 1, on a représenté les variations en fonction de la température (en K) des conductivités réduites du film obtenu dans cet exemple dénommé PANI(DEHEPSA)$_{0,5}$/DCAA et du film PANI(CSA)$_{0,5}$/MC obtenu selon l'art antérieur (référence [5]) en utilisant l'acide camphre sulfonique (CSA) comme dopant et le m-crésol (MC) comme solvant.

La conductivité réduite est la conductivité divisée par la conductivité au maximum qui dans le cas de l'échantillon testé de PANI(DEHEPSA)$_{0,5}$/DCAA est de 100 S/cm, et dans le cas de PANI(CSA)$_{0,5}$/MC est de 250 S/cm.

On remarque dans les deux cas le caractère métallique à haute température : décroissance de la conductivité quand la température augmente. Par ailleurs, on voit que le maximum se situe à une température Tmax plus faible (200 K) pour le film PANI(DEHEPSA)$_{0,5}$/DCAA que pour le film de l'art antérieur PANI(CSA)$_{0,5}$/MC où elle est de 260 K.

Ainsi, le film de polyaniline de l'invention a une conductivité de type métallique.

Sur la figure 2, on a représenté les courbes contraintes (en MPa)/allongement (ΔL/L en %) des films PANI (DEHEPSA)$_{0,5}$/DCAA et PANI(CSA)$_{0,5}$/MC, dans le cas d'essais où l'on tire régulièrement sur le film à une vitesse constante de 1 mm/min et on mesure la contrainte qu'il faut appliquer sur le film.

Dans le cas de l'invention PANI(DEHEPSA)$_{0,5}$/DCAA, on gagne plus d'un facteur 10 sur l'allongement à la rupture, ce qui montre une beaucoup plus grande plasticité du film.

**Exemple comparatif 1.**

On suit le même mode opératoire que dans l'exemple 1 pour préparer la solution sauf que l'on utilise l'acide camphre-sulfonique comme dopant à la place de l'ester di-2-éthylhexylique de l'acide 1,2-benzène dicarboxylique-4-sulpho, dans le même rapport molaire dopant/mère de polyaniline. On obtient un film de conductivité voisine mais ses propriétés mécaniques sont nettement plus mauvaises. En, particulier, il se casse au pliage.

**Exemple comparatif 2.**

On suit le même mode opératoire que dans l'exemple 1 pour préparer la solution sauf que l'on utilise le m-crésol comme solvant à la place de l'acide dichloroacétique. On obtient un film de conductivité voisine, cependant il subsiste 10 à 15 % en poids de solvant dans le film même après un pompage prolongé. Le m-crésol résiduel est à l'origine de l'odeur désagréable du film. De plus, à cause du relargage lent du solvant, les propriétés mécaniques et électriques du film changent avec le temps.

**Exemple 2**

Dans cet exemple, on prépare un film auto-supporté hautement conducteur de polyaniline dopée avec l'ester di-2-éthylhexylique de l'acide 1,2-benzène dicarboxylique-4-sulpho en suivant le même mode opératoire que dans l'exemple 1. Mais, on remplace l'acide dichloroacétique par l'acide chlorodifluoroacétique. Après coulée, on obtient un film flexible dont la conductivité, mesurée en utilisant la technique standard à quatre pointes, vaut 100 S/cm.

**Exemple 3**

Dans cet exemple, on prépare un film de composite conducteur à base de polyaniline conforme à l'invention, en utilisant comme polymère isolant le poly(méthacrylate de méthyle). On effectue la préparation de la polyaniline éméraldine base et du dopant selon le même mode opératoire que dans l'exemple 1.

<u>a) Préparation de la solution de poly(méthacrylate de méthyle) et de plastifiant dans de l'acide dichloroacétique.</u>

On prépare 5 ml de solution en mélangeant 1 g de poly(méthacrylate de méthyle) et 0,35 g de dibutylphtalate avec de l'acide dichloroacétique, par agitation vigoureuse.

<u>b) Préparation du matériau composite conducteur.</u>

On mélange 0,5 ml de la solution de poly(méthacrylate de méthyle) et de plastifiant dans de l'acide dichloroacétique avec 0,683 ml de la solution d'acide dichloroacétique contenant 2,14 mg d'éméraldine base et 5,56 mg de l'ester di-2-éthylhexylique de l'acide 1,2-benzène dicarboxylique-4-sulpho. On homogénéise le mélange par agitation vigoureuse.

On coule des films à partir de ce mélange par évaporation lente de l'acide dichloroacétique à 40°C. Les films secs ont une teneur en éméraldine base de 1,5 % en poids.

La conductivité des films ainsi obtenus, mesurée en utilisant la technique standard à quatre pointes, est de 0,55 S/cm.

**Exemple comparatif 3.**

On suit le même mode opératoire que dans l'exemple 3 pour préparer un matériau composite à partir des mêmes solutions, sauf que l'on n'introduit pas de plastifiant (dibutylphtalte). La conductivité des films obtenus ayant une teneur en éméraldine base de 1,5 % en poids, est de 0,05 S/cm, c'est-à-dire un ordre de grandeur inférieur à la valeur obtenue dans l'exemple 3.

**Références citées**

[1] : Synthetic Metals, 21, 1987, pp. 21-30.
[2] : Polymer, 34, n°20, 1993, pp. 4235-4240
[3] : Synthetic Metals, 48, 1992, pp. 91-97.

[4] : Kulszewicz-Bajer et al, Synthetic Metals, 101, 1999, pp. 713-714.
[5] : J. Chem. Phys., 103, 22, 1995, pp. 9855-9863.
[6] : Polymer Preprints, 36, 1995, pp. 396-397.
[7] : J. Phys. : Condens. Matter, 10, 1998, pp. 8293-8303.
[8] : Physical Review B, 50, 1994, pp. 13931-13941.
[9] : WO-A-98/05040
[10] : P.M. Beadle, Y. F. Nicolau, E. Banka, P. Rannou et D. Djurado dans Synthetic Metals, 95, 1998, pp. 29-45.

## Revendications

1. Composition pour la fabrication de films de polyaniline, constituée par une solution dans un solvant organique d'une polyaniline sous la forme éméraldine base et d'un dopant constitué par un acide sulfonique ou phosphonique répondant à la formule :

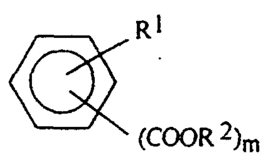 (I)

dans laquelle :

- $R^1$ représente $-SO_3H$ ou $PO_3H_2$,
- $R^2$ est un groupe alkyle linéaire ou ramifié et m est égal à 1 ou 2, ou
- $R^2$ est un groupe de formule :

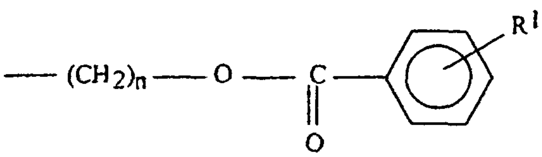 (II)

dans laquelle $R^1$ est tel que défini ci-dessus et n est un nombre entier allant de 1 à 16, et m est égal à 1, à l'exception des esters di(n-amyle), di(n-décyle), di(butoxy-2-éthyle) et di-2-(butoxy-2-éthoxy)éthyle de l'acide 5-sulfoisophtalique et des esters de l'acide 5-sulfoisophtalique et de rocanol.

2. Composition pour la fabrication d'un matériau composite conducteur comprenant :

- un solvant organique,
- une polyaniline sous la forme éméraldine base,
- un dopant constitué par un acide sulfonique ou phosphonique répondant à la formule :

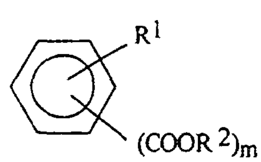 (I)

dans laquelle :

- $R^1$ représente $-SO_3H$ ou $PO_3H_2$,
- $R^2$ est un groupe alkyle linéaire ou ramifié et m est égal à 1 ou 2, ou
- $R^2$ est un groupe de formule :

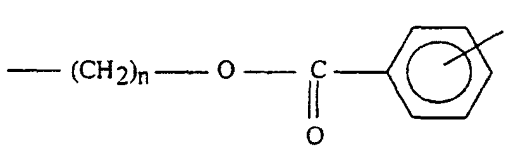

(II)

dans laquelle $R^1$ est tel que défini ci-dessus et n est un nombre entier allant de 1 à 16, et m est égal à 1,

- un polymère isolant, et
- un plastifiant du polymère isolant.

**3.** Composition selon la revendication 1 ou 2, dans laquelle le solvant est un dérivé halogéné d'un acide carboxylique de formule :

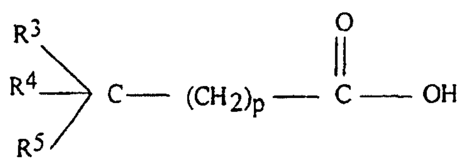

(V)

dans laquelle $R^3$, $R^4$ et $R^5$ qui peuvent être identiques ou différents, représentent H ou un atome d'halogène choisi parmi F, Cl et Br, au moins l'un des $R^3$, $R^4$ et $R^5$ représentant un atome d'halogène, et p est égal à 0,1 ou 2.

**4.** Composition selon la revendication 3, dans laquelle le solvant est choisi parmi les acides dichloroacétique, trifluoroacétique, difluoroacétique, chlorodifluoracétique, 2-chloropropionique, 2-bromobutyrique et 2,2-dichloro-propionique.

**5.** Composition selon l'une quelconque des revendications 1 à 4, dans laquelle l'acide sulfonique ou phosphonique répond à la formule (I) dans laquelle m est égal à 2.

**6.** Composition selon l'une quelconque des revendications 1 à 4, dans laquelle l'acide sulfonique ou phosphonique répond à la formule (I) dans laquelle m est égal à 1.

**7.** Composition selon l'une quelconque des revendications 1 à 4, dans laquelle l'acide sulfonique répond à la formule :

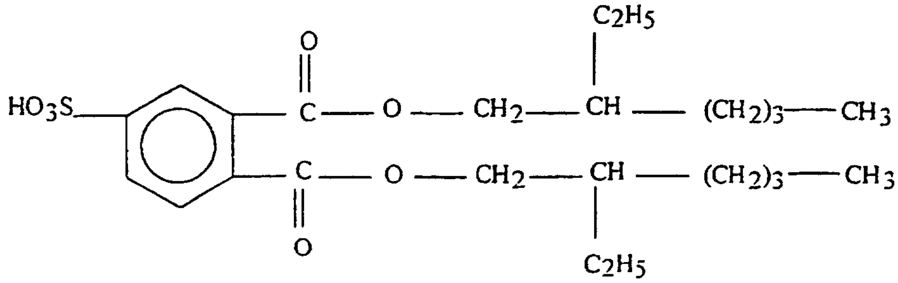

(III)

**8.** Composition selon la revendication 7, dans laquelle le solvant est l'acide dichloro-acétique.

**9.** Composition selon l'une quelconque des revendications 1 à 8, dans laquelle les teneurs en polyaniline et en dopant de la solution sont telles que le rapport molaire du dopant à la polyaniline sous la forme éméraldine base est dans la gamme de 0,4 à 0,6.

**10.** Composition selon l'une quelconque des revendications 1 à 9, dans laquelle la teneur en polyaniline de la solution est de 0,1 à 1 % en poids.

**11.** Composition selon l'une quelconque des revendications 2 à 10, dans laquelle le polymère isolant est choisi parmi le polystyrène, le poly(méthacrylate de méthyle), les polymères cellulosiques, les poly(chlorure de vinyle), les polycarbonates, les polyesters et les polyuréthanes.

**12.** Composition selon l'une quelconque des revendications 2 à 11, dans laquelle le plastifiant est choisi parmi les diesters des acides phtaliques, les diesters d'acides dicarboxyliques, et les triesters de l'acide phosphorique.

**13.** Procédé de fabrication d'un matériau composite conducteur contenant une polyaniline, **caractérisé en ce qu'**il comprend les étapes suivantes :

- préparer une composition selon l'une quelconque des revendications 2 à 12, et
- former à partir de ladite composition le matériau composite conducteur par évaporation du solvant.

**14.** Procédé selon la revendication 13, **caractérisé en ce que** l'on prépare la composition en mélangeant une première solution de polyaniline et du dopant dans le solvant à une deuxième solution dans le même solvant du polymère isolant et du plastifiant.

**15.** Matériau composite conducteur de l'électricité comprenant une matrice de polymère isolant dans laquelle sont répartis une polyaniline conductrice dopée par un acide sulfonique ou phosphonique répondant à la formule :

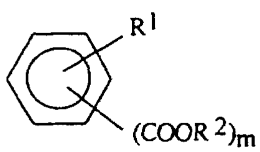

(I)

dans laquelle :

- $R^1$ représente $-SO_3H$ ou $PO_3H_2$,
- $R^2$ est un groupe alkyle linéaire ou ramifié et m est égal à 1 ou 2, ou
- $R^2$ est un groupe de formule :

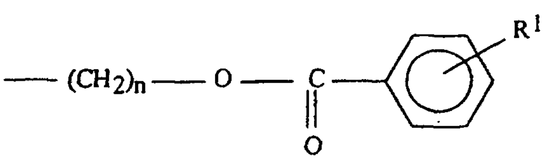

(II)

dans laquelle $R^1$ est tel que défini ci-dessus et n est un nombre entier allant de 1 à 16, et m est égal à 1, et un plastifiant du polymère isolant.

**16.** Matériau composite selon la revendication 15, dans lequel le polymère isolant est le poly(méthacrylate de méthyle).

**17.** Matériau composite selon l'une quelconque des revendications 15 et 16, qui comprend :

a) 0,06 à 10 % en poids de polyaniline et de dopant,

b) 55 à 99,9 % en poids de polymère isolant, et,
c) jusqu'à 44,94 % en poids de plastifiant du polymère isolant.

**18.** Film de polyaniline dopé par un acide sulfonique ou phosphonique répondant à la formule :

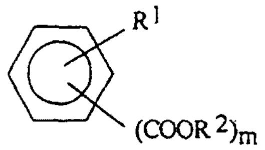

(I)

dans laquelle :

- $R^1$ représente $-SO_3H$ ou $PO_3H_2$,
- $R^2$ est un groupe alkyle linéaire ou ramifié et m est égal à 1 ou 2, ou
- $R^2$ est un groupe de formule :

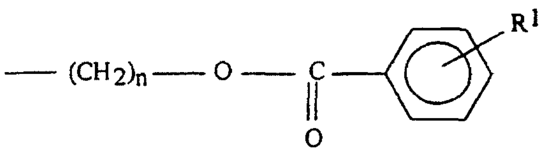

(II)

dans laquelle $R^1$ est tel que défini ci-dessus et n est un nombre entier allant de 1 à 16, et m est égal à 1, à l'exception des esters di(n-amyle), di(n-décyle), di(butoxy-2-éthyle) et di-2-(butoxy-2-éthoxy)éthyle de l'acide 5-sulfoisophtalique et des esters de l'acide 5-sulfoisophtalique et de rocanol.

**19.** Film de polyaniline selon la revendication 18, dopé par l'acide sulfonique de formule :

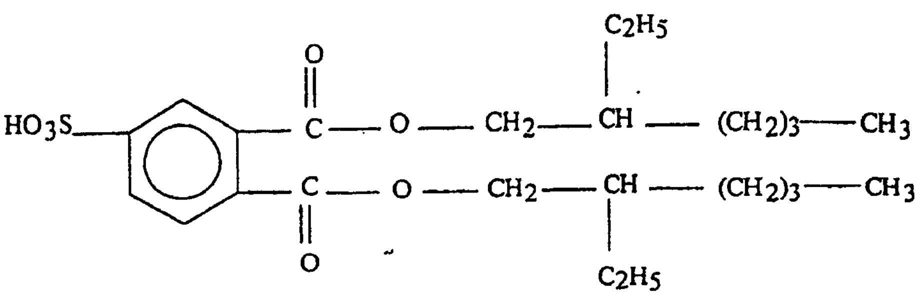

(III)

**Patentansprüche**

**1.** Zusammensetzung zur Herstellung von Polyanilinfilmen, enthaltend eine Lösung eines Polyanilins in der Emeraldin-Base-Form und eines Dotierstoffs in einem organischen Lösungsmittel, wobei der Dotierstoff eine Sulfonsäure oder Phosphonsäure enthält, die durch die folgende Formel wiedergegeben wird:

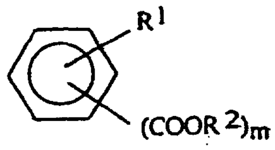

(I)

in welcher:

- $R^1$ $-SO_3H$ oder $PO_3H_2$ bedeutet,
- $R^2$ eine lineare oder verzweigte Alkylgruppe ist und m 1 oder 2 beträgt, oder
- $R^2$ eine Gruppe der Formel:

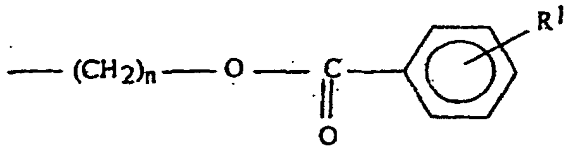

(II)

ist, in welcher $R^1$ wie vorstehend definiert ist und n eine ganze Zahl von 1 bis 16 ist und m 1 beträgt, mit Ausnahme von Di(n-amyl)-, Di(n-decyl)-, Di(butoxy-2-ethyl)-und Di-2-(butoxy-2-ethoxy)ethylestem von 5-Sulfoisophthalsäure und von Estern von 5-Sulfoisophthalsäure und von Rocanol.

**2.** Zusammensetzung zur Herstellung eines leitenden Verbundwerkstoffes, umfassend:

- ein organisches Lösungsmittel
- ein Polyanilin in der Emeraldin-Base-Form,
- einen Dotierstoff, enthaltend eine Sulfonsäure oder Phosphonsäure,

die durch die folgende Formel wiedergegeben wird:

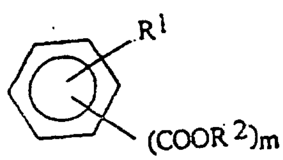

(I)

in welcher:

- $R^1$ $-SO_3H$ oder $PO_3H_2$ bedeutet,
- $R^2$ eine lineare oder verzweigte Alkylgruppe ist und m 1 oder 2 beträgt, oder
- $R^2$ eine Gruppe der Formel:

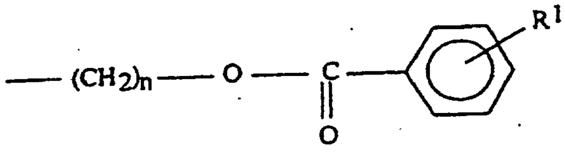

(II)

ist, in welcher $R^1$ wie vorstehend definiert ist und n eine ganze Zahl von 1 bis 16 ist und m 1 beträgt,

- ein isolierendes Polymer, und

- einen Weichmacher des isolierenden Polymers.

**3.** Zusammensetzung nach Anspruch 1 oder 2, in welcher das Lösungsmittel ein halogeniertes Derivat einer Carbonsäure der Formel:

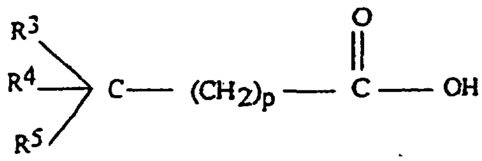 (V)

ist, in welcher $R^3$, $R^4$ und $R^5$, welche gleich oder verschieden sein können, H oder ein Halogenatom, ausgewählt aus F, Cl und Br, bedeuten, wobei wenigstens einer der Reste $R^3$, $R^4$ und $R^5$ ein Halogenatom bedeutet und p 0, 1 oder 2 beträgt.

**4.** Zusammensetzung nach Anspruch 3, in welcher das Lösungsmittel ausgewählt wird aus Dichloressigsäure, Trifluoressigsäure, Difluoressigsäure, Chlordifluoressigsäure, 2-Chlorpropionsäure, 2-Brombuttersäure und 2,2-Dichlorpropionsäure.

**5.** Zusammensetzung nach einem der Ansprüche 1 bis 4, in welcher die Sulfonsäure oder Phosphonsäure durch die Formel (I) wiedergegeben wird, in welcher m 2 beträgt.

**6.** Zusammensetzung nach einem der Ansprüche 1 bis 4, in welcher die Sulfonsäure oder Phosphonsäure durch die Formel (I) wiedergegeben wird, in welcher m 1 beträgt.

**7.** Zusammensetzung nach einem der Ansprüche 1 bis 4, in welcher die Sulfonsäure durch die folgende Formel wiedergegeben wird:

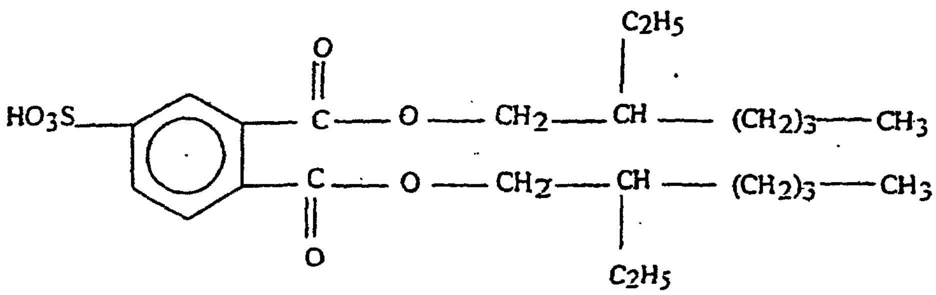 (III)

**8.** Zusammensetzung nach Anspruch 7, in welcher das Lösungsmittel Dichloressigsäure ist.

**9.** Zusammensetzung nach einem der Ansprüche 1 bis 8, in welcher die Polyanilinund Dotierstoffgehalte der Lösung so sind, dass das Molverhältnis des Dotierstoffs zu dem Polyanilin in der Emeraldin-Base-Form im Bereich von 0,4 bis 0,6 liegt.

**10.** Zusammensetzung nach einem der Ansprüche 1 bis 9, in welcher der Polyanilingehalt der Lösung 0,1 bis 1 Gew.-% beträgt.

**11.** Zusammensetzung nach einem der Ansprüche 2 bis 10, in welcher das isolierende Polymer ausgewählt wird aus Polystyrol, Poly(methylmethacrylat), Cellulosepolymeren, Polyvinylchlorid, Polycarbonaten, Polyestern und Polyurethanen.

**12.** Zusammensetzung nach einem der Ansprüche 2 bis 11, in welcher der Weichmacher ausgewählt wird aus Phthalsäurediestern, Dicarbonsäurediestern und Phosphorsäuretriestern.

**13.** Verfahren zur Herstellung eines leitenden Verbundwerkstoffs, der ein Polyanilin enthält, **dadurch gekennzeich-**

**net, dass** es die folgenden Schritte umfasst:

- Herstellen einer Zusammensetzung nach einem der Ansprüche 2 bis 12, und
- Bilden des leitenden Verbundwerkstoffs aus dieser Zusammensetzung durch Verdampfen des Lösungsmittels.

**14.** Verfahren nach Anspruch 13, **dadurch gekennzeichnet, dass** die Zusammensetzung durch Vermischen einer ersten Lösung von Polyanilin und des Dotierstoffs in dem Lösungsmittel mit einer zweiten Lösung des isolierenden Polymers und des Weichmachers in dem gleichen Lösungsmittel hergestellt wird.

**15.** Elektrizitätsleitender Verbundwerkstoff, umfassend eine Matrix aus einem isolierenden Polymer, in welcher ein leitendes Polyanilin, das durch eine Sulfonsäure oder Phosphonsäure dotiert ist, die durch die folgende Formel wiedergegeben wird:

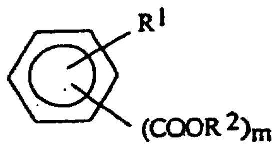

(I)

in welcher:

- $R^1$ -$SO_3H$ oder $PO_3H_2$ bedeutet,
- $R^2$ eine lineare oder verzweigte Alkylgruppe ist und m 1 oder 2 beträgt, oder
- $R^2$ eine Gruppe der Formel:

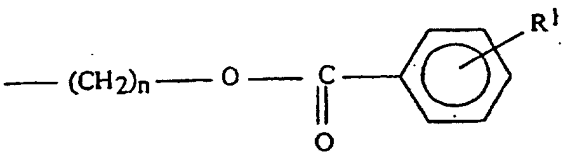

(II)

ist, in welcher $R^1$ wie vorstehend definiert ist und n eine ganze Zahl von 1 bis 16 ist und m 1 beträgt, und ein Weichmacher des isolierenden Polymers verteilt sind.

**16.** Verbundwerkstoff nach Anspruch 15, in welchem das isolierende Polymer Poly(methylmethacrylat) ist.

**17.** Verbundwerkstoff nach einem der Ansprüche 15 und 16, welcher umfasst:

a) 0,06 bis 10 Gew.-% Polyanilin und Dotierstoff,
b) 55 bis 99,9 Gew.-% isolierendes Polymer, und
c) bis zu 44,94 Gew.-% Weichmacher des isolierenden Polymers.

**18.** Polyanilinfilm, der mit einer Sulfonsäure oder Phosphonsäure dotiert ist, die durch die folgende Formel wiedergegeben wird:

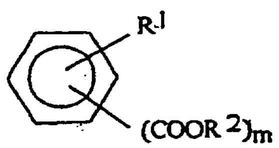

(I)

in welcher:

- $R^1$ -$SO_3H$ oder $PO_3H_2$ bedeutet,

- $R^2$ eine lineare oder verzweigte Alkylgruppe ist und m 1 oder 2 beträgt, oder
- $R^2$ eine Gruppe der Formel:

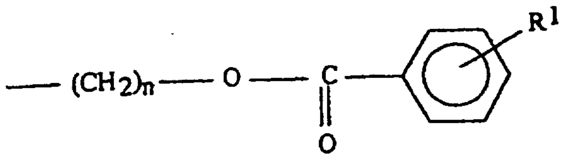

(II)

ist, in welcher $R^1$ wie vorstehend definiert ist und n eine ganze Zahl von 1 bis 16 ist und m 1 beträgt, mit Ausnahme von Di(n-amyl)-, Di(n-decyl)-, Di(butoxy-2-ethyl)-und Di-2-(butoxy-2-ethoxy)ethylestern von 5-Sulfoisophthalsäure und von Estern von 5-Sulfoisophthalsäure und von Rocanol.

**19.** Polyanilinfilm nach Anspruch 18, dotiert mit Sulfonsäure der Formel:

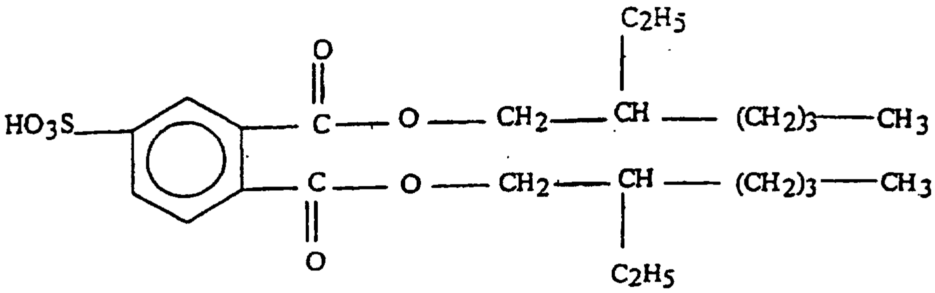

(III)

## Claims

**1.** Composition for the manufacture of polyaniline films, made up of a solution, in an organic solvent, of a polyaniline in base emeraldine form and of a dopant formed of a sulphonic or phosphonic acid, meeting the formula:

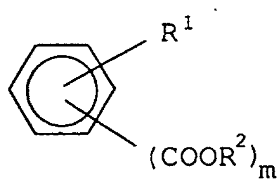

(I)

in which:

- $R^1$ represents $-SO_3H$ or $PO_3H_2$
- $R^2$ is a linear or branched alkyl group and m equals 1 or 2, or
- $R^2$ is a group having the formula:

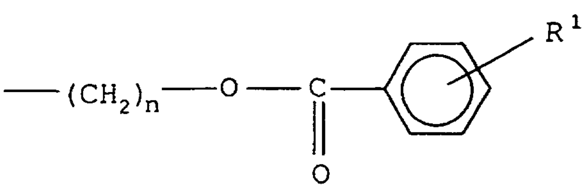

(II)

in which $R^1$ is such as defined above and n is a whole number ranging from 1 to 16, and m equals 1, with the exception of the di(n-amyl), di(n-decyl), di(butoxy-2-ethyl) and di[2-(butoxy-2-ethoxy) ethyl] esters of 5-sulphoisophtalic acid and of the esters of 5-sulphoisophtalic acid and rocanol.

**2.** Composition for the manufacture of a conductor composite material containing:

- an organic solvent,
- a polyaniline in base emeraldine form,
- a doping agent formed of a sulphonic or phosphonic acid meeting the formula:

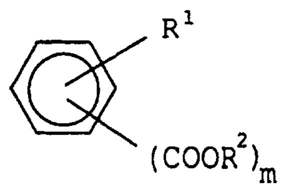

(I)

in which:

- $R^1$ represents $-SO_3H$ or $PO_3H_2$,
- $R^2$ is a linear or branched alkyl group, and m equals 1 or 2, or
- $R^2$ is a group having the formula:

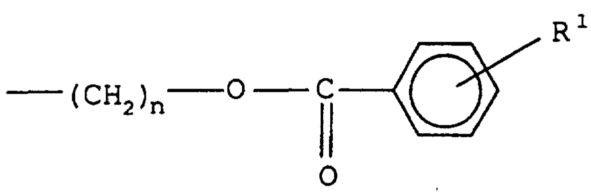

(II)

in which $R^1$ is such as defined above and n is a whole number ranging from 1 to 16, and m equals 1,

- an insulating polymer, and
- a plasticizer for the insulating polymer.

**3.** Composition according to claim 1 or 2, in which the solvent is a halogenated derivative of a carboxylic acid having the formula:

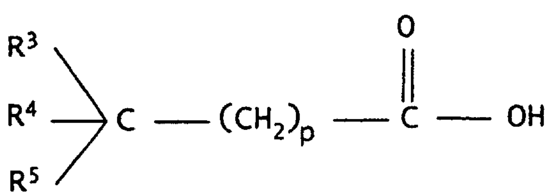

(V)

in which $R^3$, $R^4$ and $R^5$, which may be identical or different, represent H or a halogen atom chosen from among F, Cl and Br, at least one of $R^3$, $R^4$ and $R^5$ representing a halogen atom, and p equals 0.1 or 2.

**4.** Composition according to claim 3, in which the solvent is chosen from among dichloroacetic, trifluoroacetic, difluoroacetic, chlorodifluoroacetic, 2-chloropropionic, 2-bromobutyric and 2,2-dichloroproprionic acids.

**5.** Composition according to any of claims 1 to 4, in which the sulphonic or phosphonic acid meets formula (I) in which m equals 2.

**6.** Composition according to any of claims 1 to 4, in which the sulphonic or phosphonic acid meets formula (I) in which m equals 1.

**7.** Composition according to any of claims 1 to 4, in which the sulphonic acid meets the formula:

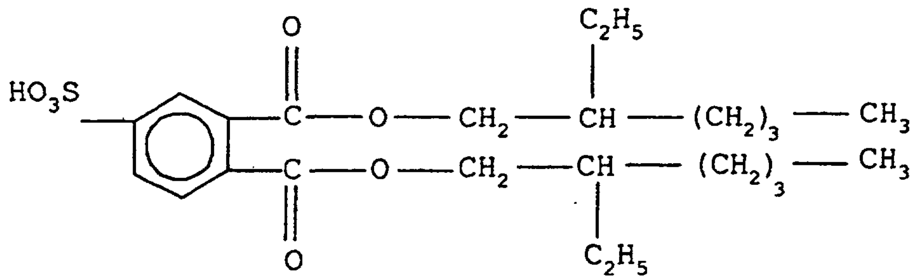

(III)

**8.** Composition according to claim 7, in which the solvent is dichloro-acetic acid.

**9.** Composition according to any of claims 1 to 8, in which the polyaniline and doping agent contents in the solution are such that the molar ratio of the doping agent to the polyaniline in base emeraldine form lies within the range of 0.4 to 0.6.

**10.** Composition according to any of claims 1 to 9, in which the polyaniline content of the solution is 0.1 to 1 % by weight.

**11.** Composition according to any of claims 2 to 10, in which the insulating polymer is chosen from among polystyrene, polymethylmethacrylate, cellulose polymers, polyvinylchloride, polycarbonates, polyesters and polyurethanes.

**12.** Composition according to any of claims 2 to 11, in which the plasticizer is chosen from among the diesters of phtalic acids, the diesters of dicarboxylic acids and the triesters of phosphoric acid.

**13.** Method for manufacturing a conductor composite material containing a polyaniline, **characterized in that** it comprises the following steps:

- preparing a composition according to any of claims 2 to 12, and
- forming the conductor composite material from said composition by evaporation of the solvent.

**14.** Method according to claim 13, **characterized in that** the composition is prepared by mixing a first solution of polyaniline and dopant in the solvent with a second solution in the same solvent of the insulating polymer and of the plasticizer.

**15.** Electricity conductive composite material containing a matrix of insulating polymer in which a conductor polyaniline is distributed doped with a sulphonic or phosphonic acid, meeting the formula:

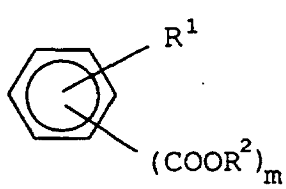

(I)

in which:

- $R^1$ represents $-SO_3H$ or $PO_3H_2$,
- $R^2$ is a linear or branched alkyl group, and m equals 1 or 2, or
- $R^2$ is a group having the formula:

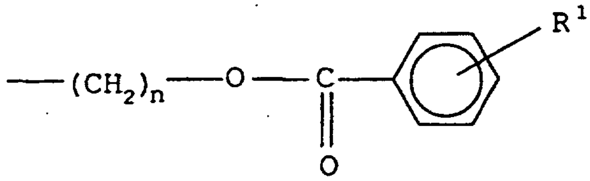

(II)

in which $R^1$ is such as defined above and n is a whole number ranging from 1 to 16, and m equals 1, and a plasticizer for the insulating polymer.

**16.** Composite material according to claim 15, in which the insulating polymer is polymethylmethacrylate.

**17.** Composite material according to either of claims 15 and 16, which contains:

a) 0.06 to 10 % by weight polyaniline and dopant,
b) 55 to 99.9 % by weight insulating polymer, and
c) up to 44.94 % by weight of plasticizer for the insulating polymer.

**18.** Polyaniline film, doped with a sulphonic or phosphonic acid, meeting the formula:

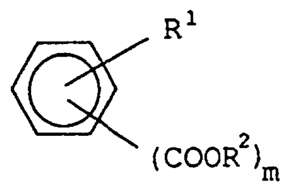

(I)

in which:

- $R^1$ represents $-SO_3H$ or $PO_3H_2$,
- $R^2$ is a linear or branched alkyl group and m equals 1 or 2, or
- $R^2$ is a group having the formula:

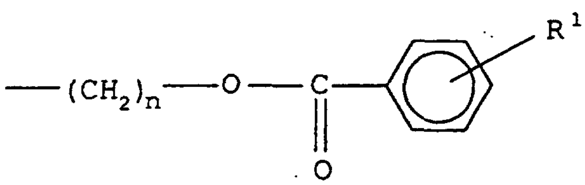

(II)

in which $R^1$ is such as defined above and n is a whole number ranging from 1 to 16, and m equals 1, with the exception of the di(n-amyl), di(n-decyl), di(butoxy-2-ethyl) and di[2-(butoxy-2-ethoxyl)ethyl] esters of 5-sulphoisophtalic acid and the esters of 5-sulphoisophtalic acid and rocanol.

**19.** Polyaniline film according to claim 18, doped with sulphonic acid having the formula:

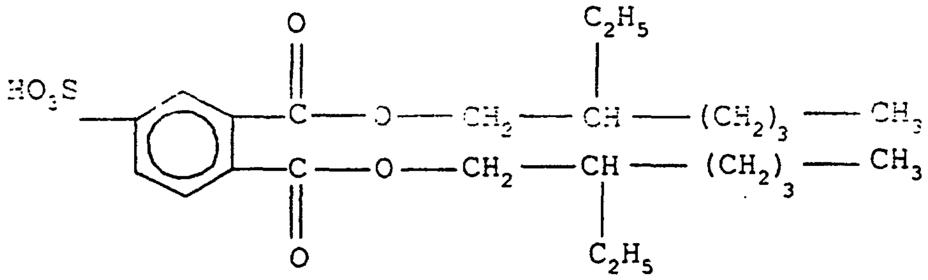 (III)

FIG. 1

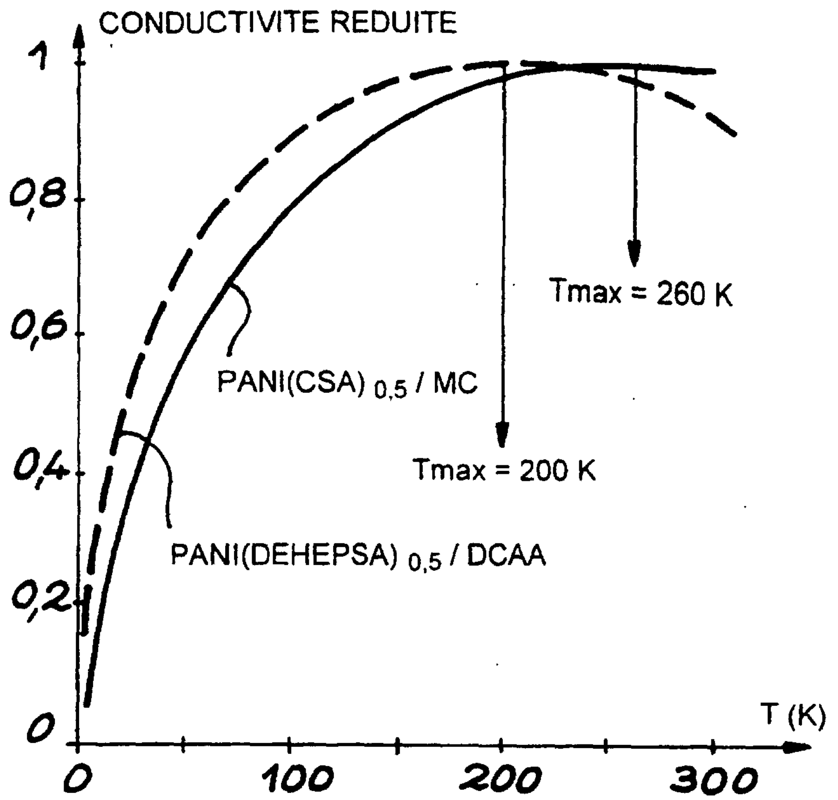
CONDUCTIVITE REDUITE
1
0,8
0,6
0,4
0,2
0
Tmax = 260 K
PANI(CSA) $_{0,5}$ / MC
Tmax = 200 K
PANI(DEHEPSA) $_{0,5}$ / DCAA
T (K)
0
100
200
300

FIG. 2

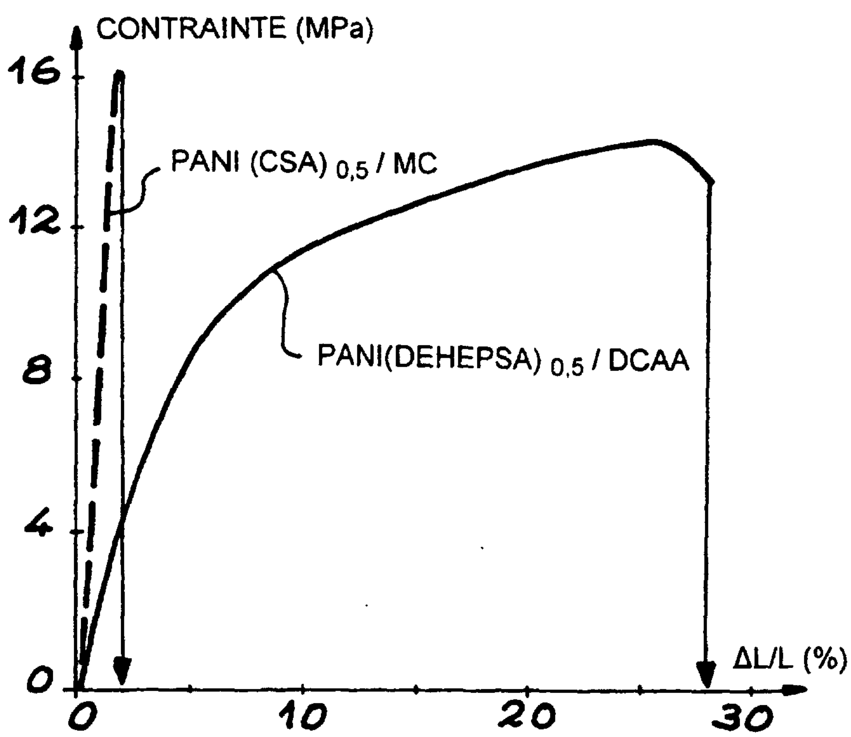
CONTRAINTE (MPa)
16
12
8
4
0
PANI (CSA) $_{0,5}$ / MC
PANI(DEHEPSA) $_{0,5}$ / DCAA
ΔL/L (%)
0
10
20
30